# EUROPÄISCHE PATENTSCHRIFT

(11) **EP 1 850 830 B1**
(45) Veröffentlichungstag und Bekanntmachung des Hinweises auf die Patenterteilung: **28.10.2009**
(21) Anmeldenummer: 06722529.2
(22) Anmeldetag: 24.02.2006
(51) Int. Cl.: A61K 9/107, A61K 47/20, A61K 47/24, A61P 19/00, A61P 35/04, A61P 39/06

(54) **SUPPOSITORIUM ZUR BINDUNG VON REAKTIVEN SAUERSTOFFVERBINDUNGEN IM KÖRPER VON MENSCHEN UND TIEREN**
SUPPOSITORY FOR BINDING OF REACTIVE OXYGEN COMPOUNDS IN HUMAN AND ANIMAL BODIES
SUPPOSITOIRE DESTINE A LIER DES COMPOSES OXYGENE REACTIFS DANS LE CORPS D'UN HOMME OU D'UN ANIMAL

(30) Priorität: 25.02.2005 DE 102005009515
(43) Veröffentlichungstag der Anmeldung: 07.11.2007
(73) Patentinhaber: Exner, Heinrich, 39291 Möckern (DE)
(72) Erfinder: Exner, Heinrich, 39291 Möckern (DE)
(74) Vertreter: Kietzmann, Manfred
(86) Internationale Anmeldenummer: PCT/DE2006/000355
(87) Internationale Veröffentlichungsnummer: WO 2006/089537

(56) Entgegenhaltungen:
- WO-A-00/50085
- WO-A-95/15768
- WO-A-02/074283

## Beschreibung

### Gebiet der Erfindung

Aus der DE 103 13 196 ist eine Silikonölemulsion zur Prophylaxe und Therapie von Erkrankungen in Folge der Bildung von reaktiven Sauerstoffradikalen bekannt. Diese Emulsion wird als Salbe oder Injektion verabreicht. Sie weist als wesentliche Bestandteile 0,01-85 Gew.-% Silikone, insbesondere Polydimethylsiloxane, 0,01-35 Gew.-% hydrophoben Emulgator mit einem HLB-Wert von 1 bis 7 und/oder einem hydrophilen Emulgator mit einem HLB-Wert von 7 bis 14 und/oder 0,01-35 Gew.-% eines Gemisches aus hydrophoben und hydrophilen Emulgator mit einem HLB-Wert von 1 bis 14, 0,1-99 Gew.-% biokompatibler Salzlösung und 0,01-40 Gew.-% Dimethylsulfoxyd auf. Nachteilig an der Applikation als Injektion, insbesondere bei einer intravenösen Injektion, ist es, dass diese ausschließlich nur durch Fachpersonal durchgeführt werden kann. Dies macht es für den Patienten notwendig, für jede Behandlung den Arzt oder ein Krankenhaus aufzusuchen. Die Applikation als Salbe zeigt eine ausschließlich lokale Wirkung und ist wenig für eine ganzkörperliche Behandlung geeignet.

Der der Erfindung zugrunde liegende Hintergrund ist der folgende. Infektionen mit entzündlichen Folgeerscheinungen können durch die Anhäufung von Sauerstoffintermediaten und deren Folgeprodukte zu einem Kollaps des Abwehrsystems - Immunparalyse - führen (SIRS, septischer Schock). Durch den Einsatz der bekannten Emulsion in größeren Dosen erfolgt eine Neutralisation dieser ungesättigten Sauerstoffintermediaten, die Immunparalyse wird gelöst und die Phagozytose der Erreger kann fortgesetzt werden.

Ähnliche Effekte treten auch bei der Schmerzbekämpfung auf. Der Schmerz, hervorgerufen durch Entzündungen, stellt die sensible Darstellung der Anhäufung dieser ungesättigten Intermediaten und ihrer Folgeprodukte dar. Durch die Applikation der bekannten Emulsion in höheren Dosen wird der Schmerz ursächlich bekämpft, indem die negativen Ladungen abgepuffert werden.

Im Zusammenhang mit der Behandlung von Erkrankungen mit der bekannten Silikonölemulsion wurden folgende Beobachtungen gemacht, die auf physiko-chemischen Grundlagen beruhen: Silikonöle sind dielektrisch und dipolar. In der bekannten Emulsion sind die Silikonöle emulgiert und weisen eine sehr große Oberfläche aus.

In den meisten Fällen erhält ein Dielektrikum seine Eigenschaften durch Polarisation. Wenn ein Dielektrikum in ein elektrisches Feld gebracht wird, richten sich die im Dielektrikum enthaltenen Dipole nach dem Feld aus. Unter Dipol versteht man allgemein die Anordnung zweier entgegengesetzter Ladungen - z.B. ein Molekül mit einem elektrisch positiven und elektrisch negativen Ende. Aufgrund dieser Polarisation steht das Dielektrikum unter Spannung und speichert Energie, die wieder abgegeben werden kann, sobald das elektrische Feld entfernt wird.

Die in der bekannten Emulsion neben Silikonöl vorhandenen dipolaren Lösungsmittel und die auf Basis von Polyoxiethylen verwendeten Emulgatoren erhöhen den Dipolcharakter der Emulsion. Die elektrostatischen Wechselwirkungen werden aufgebaut und sogenannte Van-der- Waals-Kräfte, die zwischen permanenten und induzierten Dipolen entstehen, resultieren. (D.Voet, Judith G.Voet, Charlotte W.Pratt, Biochemie S. 26 ff. 2002 Wiley-VCH Verlag, Weinheim).

Je größer die Oberfläche der dipolaren Silikonmoleküle ausgebildet ist, desto größer ist auch die Ladungsaufnahme. In Rattenpfoten-Versuchen konnte der Abtransport der Silikonöle aus der Emulsion bereits ab dem 1. Tag nach der Applikation der Emulsion beobachte werden. Nach dem 4. Tag nahm der Abtransport durch Monozyten und neutrophile Granulozyten bereits erheblich ab.

Der Dipolcharakter des nicht emulgierten Silikonöls hat auf Grund seiner geringen Oberfläche keine Bedeutung für den Abbau pathogener SauerstoffVerbindungen.

Daraus folgt, dass nur Silikonölemulsionen zum Elektronentransfer geeignet sind. Die terminale Begrenzung der bekannte Silikonölemulsion durch Abbau bzw. Verstoffwechselung der Bestandteile ist für deren klinische Effekte von großer Bedeutung.

Die bekannte Silikonölemulsion ist bereits zur Stimulierung der unspezifischen Abwehr des Immunsystems bei diversen Erkrankungen beschrieben worden. Die Dosierung lag dabei bei durchschnittlich 1 ml. Diese Dosierung reichte nach den damals vorliegenden Erkenntnissen aus, um eine gezielte Behandlung durchführen zu können und das unspezifische Immunsystem anregen zu können. Die ersten klinischen Effekte in der Behandlung der Erkrankungen traten erst nach 1 bis 3 Wochen ein.

Die Feststellung der in der bekannten Silikonölemulsion vorhandenen Van-der-Waals-Kräfte eröffnen ein völlig neues Feld für die Verwendung dieser Emulsion zur Behandlung von Krankheitsprozessen. Die durch die ungesättigten

Sauerstoffintermediaten auftretenden pathologischen Effekte und ihre Folgeprodukte können frühzeitig elektrostatisch abgepuffert werden und geben dem Krankheitsverlauf einen wesentlich prognostisch günstigeren Verlauf. Die negativen Ladungen der ungesättigten Sauerstoffintermediaten werden durch die bekannte Silikonölemulsion gepuffert. Die Neutralisation dieser Ladungen erfolgt durch den Körper selbst.

Nachteilig bei der Art der Applikation dieses Arzneimittels ist, dass die Silikone vom Körper aufgenommen und dort in Geweben und Organen eingelagert werden können. Ein Abbau der Silikone erfolgt nicht. Dies ist ein wesentlicher Nachteil.

Aus der Patentschrift DE 44 99 552 ist ein Adjuvans für Antigene sowie ein Verfahren zur Herstellung und Verwendung eines Adjuvans für Antigene bekannt.

Eine weitere Adjuvans-Emulsion beschreibt die WO-Schrift 02/074283. Diese WO-Schrift offenbart eine Emulsion bestehend aus Polydimethylsiloxanen, DMSO, einem hydrophoben Emulgator oder einem Gemisch aus einem hydrophoben und einem hydrophilen Emulgator sowie einer biokompatiblen Salzlösung. Die Emulsion wird zur Behandlung von u.a. SIRS, akuten und chronischen Erschöpfungszuständen, Tumorerkrankungen oder akuten und chronische Schmerzzuständen eingesetzt. Die Verabreichung erfolgt subkutan oder intramuskulär.

Die WO 95/15768 beschreibt ein Inkomplettes Adjuvans für Antigene wie Viren, Bakterien, Parasiten einschließlich ihrer Stoffwechselprodukte bzw. Teile der Strukturen von Viren, Bakterien und Parasiten zur Immunisierung, welches aus 0,01%-30% Polydimethylsiloxane, 0,01%-15% Komplexemulgator mit einem HLB-Wert von 9-16, 45%-99% biokompatible Salzlösung und 0,01-10% Dimethylsulfoxid, 0,0001-1% Chelatbildner besteht.

Die Verwendung einer Öl-in-Wasser-Emulsion als Arzneimittel bzw. zur Herstellung eines Arzneimittels ist der Patentschrift US 6 288 026 beschrieben.

Eine weitere Öl-in-Wasser-Emulsion zur Verwendung als Arzneimittel bzw. zur Herstellung eines Arzneimittels ist in der WO 00/50085 offenbart. Diese beschreibt eine Öl-in-Wasser-Emulsion, welche zur subkutanen oder intramusukulären Applikation hergerichtet ist und aus Polydimethylsiloxanen, einem hydrophilen Emulgator, einer biokompatiblen Salzlösung und Dimethylsulfoxid besteht. Mit der offenbarten Emulsion werden Erkrankungen wie SIRS, Viruserkrankungen wie etwa Hepatitis C und AIDS, Migräne, rheumatoide Arthritis, akute und chronische Entzündungen, akute und chronische Erschöpfungszuständen, Tumorerkrankungen oder akute und chronische Schmerzzustände behandelt.

Aufgabe der Erfindung ist es daher, eine rückstandsfreie Medikation durchführen zu können, um mögliche entzündliche Reaktionen im Körper auszuschließen.

Aus dem Stand der Technik sind nunmehr auch Suppositorien bekannt, die bei Körpertemperatur schmelzende Substanzen und Wirkstoffe enthalten. Sie sind gewöhnlich kegel-, walzen- oder torpedoartig ausgebildet und finden bei der rektalen und/oder vaginalen Applikation von Wirkstoffen Anwendung.

Derartige Wirkstoffe können gemäß EP 0 031 561 Cl umfassen:
Xanthine, Antikrebsmittel, Antibiotika, Polypeptide, Antiarrhythmika, andere kardiovaskuläre Mittel, Antidiabetika, Antiulcusmittel, Antifungusmittel, Sedativa, Diuretika, antihypertensive Mittel und Arzneimittel zur Behandlung der Parkinson' schen Krankheit.

In der DE 37 09 861 A1 ist eine silikonfreie
Suppositoriengrundmasse beschrieben, bestehend aus einer wasserunlöslichen Fettmasse und einem Anlagerungsprodukt von 5 bis 50 Mol Ethylenoxid an 1 Mol gehärtetes Rizinusöl als Emulgator, zur Herstellung von Zäpfchen (Suppositorien) mit einem Gehalt an wässrigen Wirkstofflösungen. Bevorzugt sind 0,2 bis 10 Gew.-% des Emulgators enthalten. Mit der Masse lassen sich silikonfreie Zäpfchen mit bis zu 30 Gew.- % darin dispergiertem Wasser herstellen.

Damit wird die zur Einarbeitung von Wirkstoffe, die nur in wässriger Lösung verfügbar oder verarbeitbar sind, ermöglicht. Solche Wirkstoffe können in solchen Mengen mit den Suppositoriengrundmassen verarbeitet werden, dass die dabei enthaltenen Zäpfchen bis zu 30 Gew.-% Wasser homogen verteilt, d.h. homogen dispergiert enthalten.

Der Erfindung liegt das technische Problem zugrunde, ein Verfahren zur Zubereitung einer Silikonölemulsion zur Behandlung von Infektionen (Entzündungen) hervorgerufen durch die Anhäufung von Sauerstoffintermediaten und deren Folgeprodukte ohne zusätzliche Belastung und Reizung des Organismus und Immunsystems durch Einlagerung von Silikonen anzugeben.

Zur Lösung dieses Problems lehrt die Erfindung ein Suppositorium zur rektalen und vaginalen Applikation enthaltend eine feste Silikonölemulsion, **dadurch gekennzeichnet, dass** das Suppositorium 0,01-85 Gew.-% Silikonöl und 0,01-45 Gew.-% Dimethylsulfoxyd enthält. Das Suppositorium zeichnet sich dadurch aus, dass sie sich nach der Applikation auflöst und die Silikonölemulsion freigesetzt wird, wobei Dimethylsulfoxyd (DMSO), sowie gegebenenfalls zugesetztes Glyzerin und/oder die physiologische Kochsalzlösung resorbiert und die restlichen Bestandteile des Suppositoriums aufgrund deren Molekülgröße im Darm oder Vagina verbleiben und nachfolgend restlos ausgeschieden werden.

Die Erfindung beruht auf der überraschenden Erkenntnis, dass die DMSO-haltige Silikonölemulsion während und nach dem Auflösevorgang der wasserunlöslichen Fettmasse des Suppositoriums im Darm oder der Vagina stabil und dadurch die zur Abpufferung von den Organismus schädigenden Sauerstoffintermediaten zwingend notwendige große Oberfläche erhalten bleibt. Auch ist überraschend, dass die ganzkörperliche (systemische) schmerzlindernde und entzündungshemmende Wirkung der DMSO-haltigen Silikonölemulsion erhalten bleibt, obwohl die Silikone nicht vom Körper aufgenommen werden, jedoch das DMSO vom Körper resorbiert wird.

Der erfindungsgemäße Wirkungsmechanismus des Suppositoriums beruht darauf, dass DMSO während des Auflösevorganges des Suppositoriums kontinuierlich und gleichmäßig im Darm freigesetzt wird. Dort wird es über die Darmschleimhaut vom Körper aufgenommen und über den Blutkreislauf in wenigen Minuten in alle Organsysteme transportiert. In entzündlichen Bereichen von Organen oder Bereichen mit hohen Konzentrationen ungesättigter Sauerstoffintermediate bindet DMSO die Sauerstoffintermediate und/oder leitet diese an die stabile Emulsion des Silikonöls im Darm weiter, wo die negativen Ladungen vom emulgierten Silikon aufgrund seines in der Emulsion ausgebildeten hohen Dipolcharakters gebunden, neutralisiert und über den Kot ausgeschieden werden. Die Schmerzreduzierung setzt daher innerhalb sehr kurzer Zeit, maximal von 15 Minuten, ein. DMSO weist keine Depoteffekte im Körper auf, da es neben der Ausscheidung über den Darm auch über Haut und Lunge ausgeschieden wird.

Literatur: (Pharmacology of DMSO, Stanley W. Jacob and Robert Herschier, Department of Surgery • Oregon Health Science University • Portland, Oregon 97201, 2001-2003).

Es wird herausgestellt, dass die erfindungsgemäße Wirkung von DMSO nur in Kombination mit der genannten Silikonölemulsion erreicht wird. Eine Gabe von reinem DMSO oder in Kombination mit nicht emulgierten Silikonen führt nicht zur erfindungsgemäßen hohen schmerzlindernden und entzündungshemmenden Wirkung.

Auch wird durch das erfindungsgemäße Suppositorium enthaltend eine feste DMSO-haltige Silikonölemulsion gewährleistet, dass eine langsame und gleichmäßige DMSO-Abgabe aus dem Suppositorium erfolgt. Einerseits ist dies in der Form der Emulsion an sich, andererseits durch die Zusammensetzung des Suppositoriums begründet. Die DMSO-haltige Silikonölemulsion wird beim Auflösen des wasserunlöslichen Fettmasseanteils des Suppositoriums im Darm bzw. der Vagina langsam und kontinuierlich freigesetzt, d.h. das erfindungsgemäße Suppositorium weist neben dem Depotcharakter der Silikonölemulsion einen zusätzlichen Depotcharakter auf. Durch die Wahl der Art und Anteile der Bestandteile des Suppositoriums kann die Geschwindigkeit der Freisetzungen der DMSO-haltigen Silikonölemulsion bestimmt werden. Dies ist u.a. durch die Art der gewählten wasserunlöslichen Fettmassen (Kettenlänge, Schmelzverhalten, Bindungscharakter) zu erreichen. Hierdurch kann die Dosierung der DMSO-haltigen Silikonölemulsion an das zu behandelnde Krankheitsbild angepasst werden.

Aufgrund dessen können bei gleicher Wirkungsintensität deutlich geringere Mengen an DMSO appliziert werden. Bei herkömmlichen Zubereitungen zur Behandlung von chronischen Erkrankungen beim Menschen kann DMSO in einer Dosis bis lg/kg Körpergewicht verabreicht werden. Eine toxische Wirkung tritt beim Menschen erst bei einer Zufuhr von über lg/kg KG DMSO auf (N. MLuerry et al. Drug. Intell. Clin Pharm. 18, 591 [1984}. Durch die erfindungsgemäße Silikonölemulsion wird beim Menschen die gleiche Wirkung mit einer Applikationsmenge des DMSO von 1mg/kg Körpergewicht erzielt. Bei der erfindungsgemäßen Silikonölemulsion reduziert sich somit die zu applizierende Menge an DMSO auf 1/1000 der bisher üblichen Applikationsmenge.

Vorteilhaft an der rektalen Applikation ist es, dass die primäre Leberpassage großteils umgangen wird, da die in den unteren zwei Dritteln des Rektums resorbierten Anteile der Emulsion direkt in die untere Hohlvene und nicht in die Pfortader gelangen. Die Wirkung der erfindungsgemäßen Silikonölemulsion setzt innerhalb weniger Minuten, bevorzugt nach 5 bis 15 min, im Durchschnitt nach 10 min, ein und hält nachhaltig über den gesamten Resorptionszeitraum von 2 bis 6 Stunden, durchschnittlich von 4 Stunden an.

Vorzugsweise werden wasserunlösliche Fettmassen, welche als Grundmasse des erfindungsgemäßen Suppositoriums verwendet werden, bevorzugt ausgewählt aus der Gruppe "Kakaobutter, gehärtete Pflanzenfette, Gemische von Mono, Di- und Triglyceride gesättigter Fettsäuren, Wachse" verwendet.

Die erfindungsgemäßen Emulgatoren können ausgewählt sein aus der Gruppe "Emulgatoren mit einem HLB Wert von 1 bis 14". Vorzugsweise werden Emulgatoren auf Basis von Polyoxyethylen verwendet. Diese erhöhen den Dipolcharakter der Emulsion.

Als weitere Hilfsstoffe können neben Cetylstearylalkohol, Bienenwachs, Glycerinmonostearat, Aluminiumstearat, Bentonit, Cellulosen, flüssige Triglyceride, Paraffine, hochdisperse Kieselsäure bzw. Kieselgur, Calciumcarbonat, Magnesiumoxyd, Antioxidantien dem erfindungsgemäßen Suppositorium zugesetzt werden.

Vorzugsweise besteht das Suppositorium aus 0,01-85 Gew.-% Silikonöle, insbesondere Polydimethylsiloxane, 0,01-35 Gew.-% hydrophoben Emulgator mit einem HLB-Wert von 1 bis 7 und/oder einem hydrophilen Emulgator mit einem HLB-Wert von 7 bis 14 und/oder 0,01-35 Gew.-% eines Gemisches aus hydrophoben und hydrophilen Emulgator mit einem HLB-Wert von 1 bis 14, 0,1-99 Gew.-% biokompatibler Salzlösung, 0,01-40 Gew.-% Dimethylsulfoxyd (DMSO), 0,01- 35 Gew.-% Glycerin, 0,01- 45 Gew.-% Cetylstearylalkohol, 0,01- 80 Gew.-% wasserunlösliche Fettmasse, wobei sich das Suppositorium nach der Applikation auflöst und die Emulsion freigesetzt wird, wobei Dimethylsulfoxyd (DMSO), Glyzerin sowie die physiologische Kochsalzlösung resorbiert und die restlichen Bestandteile des Suppositoriums aufgrund deren Molekülgröße im Darm oder Vagina verbleiben und nachfolgend restlos ausgeschieden werden.

In einer besonderen Ausführungsform besteht das Suppositorium aus 8,3 Gew.-% Dimeticon, 3,2 Gew.-% Polysorbat 80, 11,5 Gew.-% Cetylstearylalkohol, 8,3 Gew.-% Dimethylsulfoxyd, 9,8 Gew.-% physiologische Kochsalzlösung 9,8 Gew.-% Glycerin, 49,1 Gew.-% Kakaobutter.

Vorzugsweise wird das Suppositorium enthaltend eine feste Silikonölemulsion zur Behandlung von Schmerzzuständen eingesetzt. Insbesondere werden Schmerzzustände behandelt, die bei Erkrankungen wie bei Autoimmunerkrankungen oder Neoplasien auftreten.

Darüber hinaus wurde das erfindungsgemäße Suppositorium erfolgreich bei der Behandlung von Blockaden im Wirbelbereich eingesetzt.

Das erfindungsgemäße Suppositorium ist leicht und schmerzfrei zu applizieren, wodurch die Annahme des Suppositoriums insbesondere bei Kindern und Schmerzpatienten deutlich verbessert wird. Durch die Form als Suppositorium wird eine ganzkörperliche Behandlung des Patienten zu Hause ermöglicht, dadurch dass sich der Patient das Suppositorium selbst verabreichen oder beispielsweise durch ein Familienmitglied verabreichen lassen kann. Auch sind bei langzeitlichen Therapien weniger Reizzustände als bei wiederkehrenden, insbesondere bei intramuskulären Injektionen zu rechnen, da die Silikonöle nach ihrer Auflösung als Emulsion im Darm aus dem Körper ausgeschieden werden.

Bei der Silikonölemulsion kann es sich um eine Öl-in-Wasser, Wasser-in-Öl als auch um eine doppelte Emulsion handeln.

Im Dickdarm verlaufen bis zu 90% des Stofftransportes nicht durch die Enterozyten hindurch, sondern über den parazellulären Weg. Die Tight Junctions im Kolon und Rektum sind frei durchgängig für Moleküle bis zu einem Durchmesser von maximal 0,2 - 0,25 nm, jedoch weniger durchlässig oder undurchlässig für hochmolekulare Stoffe. Ein indirektes Maß zur Bestimmung der Molekülgröße beim Silikonöl ist die Bestimmung der kinematischen Viskosität. Es wurde festgestellt, dass die kinematische Viskosität des Silikonöls nach PharmEUR mindestens 50 mm² • s^{"1} betragen muss, um eine Resorption auszuschließen. In einer besonderen Ausführungsform kann das erfindungsgemäße Suppositorium mit wasserlöslichen und/oder fettlöslichen Wirkstoffen kombiniert werden. Vorzugsweise kann das erfindungsgemäße Suppositorium einen oder mehrere wasserlösliche Wirkstoffe der Gruppe "Antibiotika, Wasserstoffperoxid, Schmerzmittel und wasserlösliche Vitamine,, enthalten.

Ebenso ermöglicht das erfindungsgemäße Suppositorium die Applikation von fettlöslichen Wirkstoffen. Fettlösliche Wirkstoffe können fettlösliche Vitamine sein.

Ein besonderer Aspekt der Erfindung ist die Kombination von einem oder mehreren wasserlöslichen und einem oder mehreren fettlöslichen Wirkstoffen in einem Suppositorium.

In einer besonderen Ausführungsform kann das Suppositorium zur Behandlung von Genitalerkrankungen, wobei das Suppositorium zusätzlich zu den genannten Wirkstoffen oder als alleinigen Wirkstoff eine wässrige Wasserstoffperoxydlösung enthalten kann, verwendet werden. Die Konzentration der Wasserstoffperoxydlösung beträgt hierbei 1 Gew.-% bis 4,5 Gew.-%, vorzugsweise 2,5 Gew.-%. Hierdurch wird die Behandlung von Scheidenschleimhauterkrankungen wie "Candidiosen, virale Infektionen, bakterielle Infektionen" möglich. Die Erfindung lehrt des Weiteren ein Verfahren zur Herstellung eines erfindungsgemäßen Suppositoriums aufweisend mindestens die Verfahrensschritte: Erwärmung der gesamten Rezeptur im Warmbad auf 60 - 99°C, vorzugsweise 85°C, Homogenisieren der Suppositoriumsmasse mit 12000 bis 14000 U/min, vorzugsweise mit 13.000 U/min und Abfüllen der Suppositoriumsmasse.

In einer bevorzugten Ausführungsform wird das erfindungsgemäße Suppositorium nach dem folgenden Verfahren hergestellt:
Alle Bestandteile des erfindungsgemäßen Suppositoriums werden im Wasserbad geschmolzen und auf über 80°C aufgewärmt. Danach erfolgt die Homogenisierung mit einem Rührgerät mit 12000 U/min. Die Masse wird dadurch pasteus und kann anschließend sofort in die Gussformen abgefüllt werden. Im Kühlschrank oder bei Zimmertemperatur erfolgt die Aushärtung innerhalb von 12 Stunden.

Ein unerwarteter positiver Effekt dieses
Herstellungsverfahrens ist, dass das feste Suppositorium in einem Schritt hergestellt werden kann. Die Silikonölemulsion bildet sich bei gleichzeitiger Anwesenheit der wasserunlösliche Fettmasse stabil aus. Ein weiterer Vorteil des erfindungsgemäßen Verfahrens ist, dass sich die Suppositoriumsmasse während der Homogenisierung von selbst abkühlt. Eine zusätzliche energetisch aufwendige Abkühlung der Suppositoriumsmasse ist somit nicht notwendig, wodurch das erfindungsgemäße Verfahren gegenüber bekannten Verfahren zur Herstellung von festen Zubereitungen zur rektalen, vaginalen oder oralen Applikation wirtschaftlich vorteilhaft ist.

Im Folgenden wird die Erfindung an bevorzugten Ausführungsformen näher erläutert.

### Beispiele:

H.S. *1965 weiblich: Morbus Bechterev seit über 10 Jahren. Behandlung der chronischen Schmerzen mit Celebrex. Krankheit schreitet fort und kann auch durch Celebrex nicht wesentlich beeinflusst werden. Die Nebenwirkungen von Celebrex machen der Patientin deutlich zu schaffen. Nach einer Woche Behandlung mit dem erfindungsgemäßen Suppositoriums bei täglicher Applikation verbesserte sich bereits der Allgemeinzustand und nach 4 Wochen Behandlung konnte Celebrex abgesetzt werden.

H.A. *1964 weiblich: Diagnose Mammatumor nach OP, Chemotherapiezyklen und Bestrahlung Patientin erhielt die erfindungsgemäßen Suppositorien bei täglicher Applikation zur Verbesserung der Lebensqualität. Nach wenigen Tagen gingen bereits die therapiebedingten Gliederschmerzen zurück und die Patientin nahm wieder regen Anteil am Leben.

R.B. *1955 männlich: Diagnose: Wirbelkörperblockierung Lendenwirbelbereich

Patient hatte sich bei Fußbodenlegearbeiten eine Wirbelblockierung im Lendenwirbelbereich zugezogen und konnte nicht mehr gerade laufen. Bereits nach der Applikation von einem erfindungsgemäßen Suppositorium konnte er seine Arbeit wieder fortsetzen.

S.J. *1955 weiblich: Diagnose Fatique Syndrom.
Patientin mit typischen Erschöpfungszeichen wie Schlaflosigkeit, Konzentrationsschwäche, subfebrile Temperatur und allgemeine Teilnahmslosigkeit. Nach der Behandlung mit einem erfindungsgemäßen Suppositorium kehrte nach kurzer Zeit ihr Wohlbefinden zurück. Eine Weiterbehandlung erfolgt nach Bedarf im wöchentlichen Abstand.

P. K. *1969 weiblich: Diagnose Migräneanfall.
Zur Behandlung wurden im Abstand von einer Stunde zwei des erfindungsgemäßen Suppositorium appliziert. Danach war die Patientin beschwerdefrei.

## Patentansprüche

1. Suppositorium zur rektalen und vaginalen Applikation enthaltend eine feste Silikonölemulsion, wobei das Suppositorium 0,01-85 Gew.-% Dimeticon und 0,01-45 Gew.-% Dimethylsulfoxyd enthält.

2. Suppositorium nach Anspruch 1, **dadurch gekennzeichnet, dass** das Suppositorium besteht aus 0,01-85 Gew.-% Dimeticon, 0,01-35 Gew.-% hydrophoben Emulgator mit einem HLB-Wert von 1 bis 7 und/oder einem hydrophilen Emulgator mit einem HLB-Wert von 7 bis 14 und/oder 0,01-35 Gew.-% eines Gemisches aus hydrophoben und hydrophilen Emulgator mit einem HLB-Wert von 1 bis 14,
0,1-99 Ges.-% biokompatibler Salzlösung,
0,01-40 Gew.-% Dimethylsulfoxyd,
0,01- 35 Gew.-% Glycerin,
0,01- 45 Gew.-% Cetylstearylalkohol,
0,01-85 Gew.-% wasserunlösliche Fettmasse.

3. Suppositorium nach einem der Ansprüche 1 bis 2, **dadurch gekennzeichnet, dass** das Suppositorium besteht aus
| | |
|---|---|
| 8,3 Gew.-% | Dimeticon, |
| 3,2 Gew.-% | Polysorbat 80, |
| 11,5 Gew.-% | Cetylstearylalkohol, |
| 8,3 Gew.-% | Dimethylsulfoxyd, |
| 9,8 Gew.-% | physiologische Kochsalzlösung, |
| 9,8 Gew.-% | Glycerin, |
| 49,1 Gew.-% | Kakaobutter. |

4. Suppositorium nach einem der Ansprüche 1 bis 3, **dadurch gekennzeichnet, dass**
das Dimeticon eine kinematische Viskosität von größer 50 mm² s⁻¹ aufweist.

5. Suppositorium nach einem der Ansprüche 1 bis 4, **dadurch gekennzeichnet, dass**
das Suppositorium über einen Zeitraum von 2 - 6 Stunden resorbierbar ist.

6. Suppositorium nach Anspruch 5**, dadurch gekennzeichnet, dass**
das Suppositorium über einen Zeitraum von 4 Stunden resorbierbar ist.

7. Suppositorium nach einem der Ansprüche 1 bis 6, **dadurch gekennzeichnet, dass**
das Suppositorium mit wasserlöslichen und/oder fettlöslichen Wirkstoffen kombiniert ist.

8. Suppositorium nach Anspruch 7, **dadurch gekennzeichnet, dass**
die wasserlöslichen Wirkstoffe ausgewählt sind aus der Gruppe bestehend aus "Antibiotika, Schmerzmittel, Vitaminen, Wasserstoffperoxid".

9. Suppositorium nach Anspruch 7, **dadurch gekennzeichnet, dass**
die fettlöslichen Wirkstoffe fettlösliche Vitamine sind.

10. Suppositorium nach einem der Ansprüche 1 bis 9 zur Behandlung von Autoimmunerkrankungen, Neoplasien, Blockaden im Wirbelbereich.

11. Suppositorium nach einem der Ansprüche 1 bis 9 zur Behandlung von Genitalerkrankungen, wobei das Suppositorium eine wässrige Wasserstoffperoxydlösung enthält.

12. Verwendung des Suppositoriums nach einem der Ansprüche 1 bis 9 zur Herstellung eines Arzneimittels zur Behandlung von Autoimmunerkrankungen, Neoplasien, Blockaden im Wirbelbereich.

13. Verwendung des Suppositoriums nach einem der Ansprüche 1 bis 9 zur Herstellung eines Arzneimittels zur Behandlung von Genitalerkrankungen, wobei die Zubereitung eine wässrige Wasserstoffperoxydlösung enthält.

14. Verfahren zur Herstellung eines Suppositoriums nach einem der Ansprüche 1 bis 11 enthaltend eine feste Silikonölemulsion aufweisend mindestens die Verfahrensschritte:
- Erwärmung aller Bestandteile des Suppositoriums im Warmbad auf 60 - 99°C,
- Homogenisieren mit 12000 bis 14000 U/min, vorzugsweise mit 13000 U/min,
- Abfüllen der Suppositoriumsmasse in Gussformen.

## Claims

1. Suppository for rectal or vaginal application containing a solid silicone oil emulsion wherein the suppository contains 0.01-85 percent per weight Dimeticone and 0.01-45 percent per weight dimethylsulfoxide.

2. Suppository according to Claim 1, **characterised in that** the suppository consists of 0.01-85 percent by weight silicone, in particular polydimethylsiloxane, 0.01-35 percent by weight hydrophobic emulsifying agent with an HLB value from 1 to 7 and/or a hydrophilic emulsifying agent with an HLB value from 7 to 14 and/or 0.01-35 percent by weight of a mixture of hydrophobic and hydrophilic emulsifying agent with an HLB value from 1 to 14,
0.1-99 percent by weight bio-compatible saline solution,
0.01-40 percent by weight dimethylsulfoxide,
0.01-35 percent by weight glycerol,
0.01-45 percent by weight cetylic stearylic alcohol,
0.01-85 percent by weight water-insoluble fat mass.

3. Suppository according to any of the Claims 1 to 2, **characterised in that** the suppository consists of
| | |
|---|---|
| 8.3 percent by weight | dimethicone |
| 3.2 percent by weight | polysorbate 80 |
| 11.5 percent by weight | cetylic stearylic alcohol |
| 8.3 percent by weight | dimethylsulfoxide |
| 9.8 percent by weight | physiological saline solution |
| 9.8 percent by weight | glycerol |
| 49.1 percent by weight | cocoa butter. |

4. Suppository according to any of the Claims 1 to 3, **characterised in that** the dimeticone has a kinematic viscosity of more than 50 mm² · s⁻¹.

5. Suppository according to any of the Claims 1 to 4, **characterised in that** the suppository is absorbed over a period of 2 - 6 hours.

6. Suppository according to claim 5, **characterised in that** the suppository is absorbed over a period of 4 hours.

7. Suppository according to any of the Claims 1 to 6, **characterised in that** the suppository is combined with water-soluble and/or fat-soluble active ingredients.

8. Suppository according to Claim 7, **characterised in that** the water-soluble active ingredients are selected from the group consisting of "antibiotics, analgesics, vitamins, hydrogen peroxide".

9. Suppository according to Claim 7, **characterised in that** the fat-soluble active ingredients are fat-soluble vitamins.

10. Suppository according to any of the Claims 1 to 9 for the treatment of auto-immune diseases, neoplasias, blockades in the area of vertebrae.

11. Suppository according to any of the Claims 1 to 9 for the treatment of diseases of the genital organs with the suppository containing an aqueous hydrogen peroxide solution.

12. Use of the suppository according to any of the Claims 1 to 9 for the production of a medicament for the treatment of auto-immune diseases, neoplasias, blockades in the area of vertebrae.

13. Use of the suppository according to any of the Claims 1 to 9 for the production of a medicament for the treatment of diseases of the genital organs with the suppository containing an aqueous hydrogen peroxide solution.

14. Method for the production of a suppository according to any of the Claims 1 to 11 containing a solid silicone oil emulsion and comprising at least the following steps:
- heating of all components of the preparation in a hot bath to 60 to 99° C,
- homogenisation at 12,000 to 14,000 rev/min, preferably at 13,000 rev/min,
- filling out of the suppository mass in moulds.

## Revendications

1. Suppositoire pour application rectale ou vaginale contenant une émulsion d'huile silicone solide, le suppositoire contenant 0,01-85 %-poids de diméticone et 0,01-45 %-poids de diméthylsulfoxyde.

2. Suppositoire selon la revendication 1, **caractérisé en ce que** le suppositoire se compose de 0,01-85 %-poids de diméticone, de 0,01-35 %-poids d'émulsifiant hydrophobe avec une valeur HLB de 1 à 7 et/ou d'un émulsifiant hydrophile avec une valeur HLB de 7 à 14 et/ou de 0,01-35 %-poids d'un mélange d'émulsifiant hydrophobe et hydrophile avec une valeur HLB de 1 à 14,
0,1-99 %-poids de solution saline biocompatible,
0,01-40 %-poids de diméthylsulfoxyde,
0,01-35 %-poids de glycérine,
0,01-45 %-poids d'alcool cétylstéarylique,
0,01-85 %-poids de masse de graisse insoluble dans l'eau.

3. Suppositoire selon une des revendications 1 à 2, **caractérisé en ce que** le suppositoire se compose de
| | |
|---|---|
| 8,3 %-poids | de diméticone, |
| 3,2 %-poids | de polysorbate 80, |
| 11,5 %-poids | d'alcool cétylstéarylique, |
| 8,3 %-poids | de diméthylsulfoxyde, |
| 9,8 %-poids | de solution de sel de cuisine physiologique, |
| 9,8 %-poids | de glycérine, |
| 49,1 %-poids | de beurre de cacao. |

4. Suppositoire selon une des revendications 1 à 3, **caractérisé en ce que**
le diméticone présente une viscosité cinématique supérieure à 50 mm² · s⁻¹.

5. Suppositoire selon une des revendications 1 à 4, **caractérisé en ce que** le suppositoire est résorbable en une période de 2 - 6 heures.

6. Suppositoire selon la revendication 5**, caractérisé en ce que** le suppositoire est résorbable en une période de 4 heures.

7. Suppositoire selon une des revendications 1 à 6, **caractérisé en ce que**
le suppositoire est combiné avec des constituants actifs solubles dans l'eau et/ou solubles dans les graisses.

8. Suppositoire selon la revendication 7, **caractérisé en ce que**
les constituants actifs solubles dans l'eau sont choisis dans le groupe constitué de "antibiotiques, analgésiques, vitamines, peroxyde d'hydrogène".

9. Suppositoire selon la revendication 7, **caractérisé en ce que**
les constituants actifs solubles dans les graisses sont des vitamines solubles dans les graisses.

10. Suppositoire selon une des revendications 1 à 9 pour le traitement de maladies auto-immunes, de néoplasies, de blocages dans la zone vertébrale.

11. Suppositoire de la préparation selon une des revendications 1 à 9 pour le traitement de maladies génitales, le suppositoire contenant une solution aqueuse de peroxyde d'hydrogène.

12. Utilisation du suppositoire selon une des revendications 1 à 9 pour la fabrication d'un médicament pour le traitement de maladies auto-immunes, de néoplasies, de blocages dans la zone vertébrale.

13. Utilisation du suppositoire selon une des revendications 1 à 9 pour la fabrication d'un médicament pour le traitement de maladies génitales, le suppositoire contenant une solution aqueuse de peroxyde d'hydrogène.

14. Procédé de fabrication d'un suppositoire selon une des revendications 1 à 11, contenant une émulsion solide d'huile de silicone présentant au moins les étapes de procédés :
- chauffage de tous les composants du suppositoire dans le bain chaud à 60 - 99°C,
- homogénéisation à 12 000 à 14 000 tr/min, de préférence à 13 000 tr/min,
- transvasement de la masse du suppositoire dans des moules.
